# EUROPEAN PATENT SPECIFICATION

(11) **EP 1 470 151 B1**
(45) Date of publication and mention of the grant of the patent: **30.12.2009**
(21) Application number: 03702441.1
(22) Date of filing: 14.01.2003
(51) Int. Cl.: C07J 51/00, C07J 1/00, C07J 5/00

(54) **PROCESS FOR THE PREPARATION OF 7-ALPHA-METHYLSTEROIDS**
VERFAHREN ZUR HERSTELLUNG VON 7-ALPHA-METHYLSTEROIDE
PROCEDE DE PREPARATION DE 7-ALPHA-METHYLSTEROIDES

(30) Priority: 21.01.2002 EP 02075230
(43) Date of publication of application: 27.10.2004
(73) Proprietor: N.V. Organon, 5349 AB Oss (NL)
(72) Inventor: STOELWINDER, J., NL-5340 BH Oss (NL); OSTENDORF, M., NL-5340 BH Oss (NL); BUGGENUM, Van P.A.M., NL-5340 BH Oss (NL)
(74) Representative: Kraak, Hajo
(86) International application number: PCT/EP2003/000339
(87) International publication number: WO 2003/059931

(56) References cited:
- WO-A-00/59920
- WO-A-01/05806
- WO-A-01/40255
- WO-A-01/58919
- WO-A-02/10188
- US-A- 3 341 557
- CAMPBELL, J. ALLAN ET AL: "7.alpha.-Methyl-19-norsteroids;a new class of potent anabolic and androgenic hormones" STEROIDS (1963), 1, 317-24, XP002204811
- J. A. CAMPBELL ET AL: "The Synthesis of Some 7.alpha.- and 7.beta.-Methyl Steroid Hormones" JOURNAL OF THE AMERICAN CHEMICAL SOCIETY., vol. 81, 1959, pages 4069-4074, XP002204812 AMERICAN CHEMICAL SOCIETY, WASHINGTON, DC., US ISSN: 0002-7863
- NICKISCH, KLAUS ET AL: "Stereoselective synthesis of 7.alpha.-allyl- and 7.alpha.-propylsteroids" TETRAHEDRON LETT. (1988), 29(13), 1533-6, XP002204813

## Description

The invention relates to a new process for the preparation of 7α-methylsteroids.

7α-Methylsteroids represent an important class of pharmacologically active compounds. One example of a steroid with a methylgroup at the 7-position is (7α,17α)-17-hydroxy-7-methyl-19-norpregn-5(10)-en-20-yn-3-one (Tibolone) which is the active component of Livial®, used as hormone replacement therapy in the treatment of menopausal complaints. Tibolone is a compound exerting tissue-specific hormonal activity. In laboratory and clinical studies, Tibolone exerts estrogenic effects on the thermoregulatory system, vaginal tissue and bone, but does not display estrogenic activity on breast or the endometrium. Tibolone's promising effects on bone are also currently under investigation for the prevention of osteoporosis in women who are likely to develop this condition. *(1)* Pavlov, P.W., et al. Gynecol. Endocrinol. 1999;13: 230-237, *2)* Tibolone (Livial). A new steroid for the menopause. Drug Ther. Bull. 1991;29:77-8, *3*) Moore R.A. Systematic and economical review for Livial. In: Rymer J (Ed.), Livial: A review of clinical studies. Br.J.Gynaecol. 1999;106 (3 Suppl. 19) 1)

Another pharmacological important class of 7α-methylsteroids are 7α-methyl-19-nortestosterone (MENT^{™}) and the related esters at the 17-position (WO99/67271), which are synthetic androgens being developed for hormone replacement therapy and male fertility control. Studies have shown MENT^{™} to be more potent than the male hormone testosterone in maintaining muscle mass, potency, and libido and in its ability to suppress sperm production. Also, MENT^{™} does not enlarge the prostate as much as testosterone does, which may result in safer medical use. (*1*) Ogawa, S., et al 1996; 30:74-84. Hormones and Behavior, 2) Robbins, A., et al. Society for Neuroscience Abstracts 1994;20 (part 1):376, 3) Sundaram, K., et al. Annals of Medicine 1993;25:199-205, *4*) Morali, G. et al. Biology of Reproduction 1993;49:577-581).
A third example of a potential interesting steroid with a methylgroup at the 7-position is 3-hydroxy-7a-methyl-21-[2'-methoxy-4'-(diethylaminomethyl)-phenoxy]-19-norpregna-1,3,5(10)triene citrate (SR 16234), which is a Selective Estrogen Receptor Modulator (SERM) which has been found to have potent antitumor activity with tissue selective properties, and complete antagonist-antiestrogenic activity in human breast tumor cells. (1) WO 01/58919 A2, 2) WO 99/33859 A2,3) US 6,281,205 B1,4) US 20020032180 A1).
From a synthetic point of view, the stereoselective introduction of the substituents at C-7 constitutes the key transformation in the assembly of representatives from this important class of steroids. According to literature, the introduction of an alkyl group in the 7-position of a steroid backbone is generally accomplished by a) cuprous chloride or cupric acetate catalyzed 1,6-conjugate addition of alkylmagnesiumhalogens to 4,6-unsaturated 3-ketosteroids, or b) by conjugate addition of copper-lithiumalkyl-reagents to 4,6-unsaturated 3-ketosteroids. However, in general these methods yield mixtures of 7α- and 7β-alkylsteroids (*1)* Modi, S.P. et al, J. Org. Chem. 1989;54: 2317-2321, *2)* Grunwell, J. F. Steroids, 1976;27: 6, 759. *3)* Campbell, J.A. et al., J.Am.Chem.Soc. 1959;81: 4069, *4)* Grunwell, J.F., et al., Steroids 1976;27: 750, *5)* US patent 3,798,213, 6) van Vliet N.P., et al. Recl.Trav.Chim.Pays-Bas 1986; 105:111) in α,β-ratios ranging from 1.5:1 to 9:1. Isolation of the pharmacological interesting 7α-isomers from the accompanying undesirable 7β-isomers, which are less effective enzyme inhibitors (O'Reilly, J.M. et al. J. Med. Chem 1995;38: 2842) only can be achieved by chromatographic separation, or by laborious work-up procedures by repetitive recrystallization. Both operations decrease the yield of the desired 7α-isomer significantly.
In WO 01/58919, a 4,6-unsaturated 3-ketosteroid is alkylated at the 7-position with methyllithium in the presence of lithium bromide. In this case it is reported that the stereoselectivity of this 7-alkylation increases after protecting the extant hydroxylgroup with a THP-ether, allegedly by complex formation with the lithium in favor of attack from the α-face of the steroid. Despite the increase in selectivity, unfortunately in this last method the high reactivity of the acetate protecting group with the methyllithium is reflected in only a moderate yield of 67% of the 7α-methylsteroid.
Alternative methods for the introduction of 7-alkylgroups have been developed, but are not generally applicable. Only a limited selection of alkylgroups can be introduced, the method is limited to the synthesis of steroids with an aromatic A-ring, or additional reaction steps are required, making these methods generally inefficient. For example: 7α-substituted estradiols have been prepared by conversion of 6-ketoestradiols into 6-(phenylsulfonyl)-6-dehydroestradiol, which undergoes conjugate addition of organolithium reagents to the C-7-position, followed by additional steps to remove the sulfone group. (Künzer, H. et al. Tetrahedron Lett. 1994;35: 11, 1691) In a more recent approach 7α-substituted estradiols have been prepared by alkylation of 6-ketoestradiols, followed by deoxygenation and deprotection with boron trifluoride etherate and triethylsilane. (Tedesco, R. et al. Tetrahedron Lett. 1997;3 8: 46, 7997). In WO 01/58919, a 6-ketoestradiol is alkylated at the 7-position by reaction with methyl iodide in the presence of lithium diisopropylamine, followed by catalytic removal of the 6-ketone using hydrogen and platinum and palladium; reported is that the, stereoselectivity of this 7-alkylation increases after protection of the extant hydroxylgroup with a THP-ether, allegedly by steric hindrance from the β-face of the steroid. In DE 4,418,828 A it is described that 7α-methylestradiols were prepared by a sequence of reactions from 8,9-unsaturated estradiols with formaldehyde in the presence of Lewis acids, followed by catalytic hydrogenation, tosylation, and reduction. In EP 0,262,201 B1 the preparation of 7α-propylsteroids is described from 4,6-unsaturated 3-ketosteroids by a Sakurai reaction of allyltrialkylsilanes or allyltrialkyltin compounds in the presence of Lewis acids, followed by a selective hydrogenation with tris(triphenylphosphine)rhodium(I)-chloride (Wilkinson's catalysts). (see also Nickisch, K; Laurent, H. Tetrahedron Lett. 1988;29: 13, 1533). Therefore, a major challenge in the synthesis of 7-alkylsteroids is control of the diastereoselectivity of the copper mediated 1,6-conjugate addition, which is the most straightforward method of introduction for 7-alkylgroups.

According to the present invention, a process has now been found for the preparation of 7α-methyl steroids of the formula I 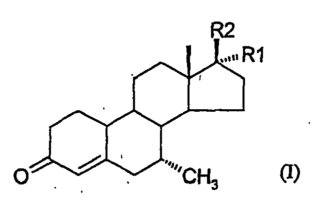
wherein R1 is hydrogen, methyl or C≡CH,
R2 is (CH₂)ₙOH, wherein n is 0, 1 or 2;
by a copper mediated 1,6-conjugate addition of a Grignard reagent CH₃MgX, X being a halogen (Cl, Br or I), to the 4,6-unsaturated 3-ketosteroid of formula II, 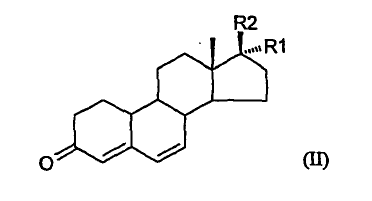 wherein R1 and R2 are as previously defined, comprising protecting the hydroxy group of the steroid of formula II with a trialkylsilyl group, followed by treating the hydroxy protected steroid with the Grignard reagent.

The process of this invention results in a major increase in solubility of the steroid substrate, opens up the possibility to increase the concentration of steroid substrates in said process (a range of concentrations is possible, but preferably the concentration of the steroid is 0.1 to 0.3 molar), and surprisingly shows a markedly improved stereoselectivity in favor of the desired 7α-isomer. The levels of the unwanted 7β-isomer are decreased to levels below 2.5%, which is a more than sixfold improvement in selectivity (from approximately 6:1 to 39: 1).

Although the trialkylsilyl protection of a hydroxy group in a process wherein a 7α-ethyl group was introduced has been described previously (WO 01105806), the reported selectivity was only 85:15 (α:β).

Preferably, R1 is hydrogen, methyl or C≡CH and R2 is OH; or R1 is hydrogen and R2 is (CH₂)₂OH. More preferably, when R2 is OH, R1 is hydrogen or C≡CH.
The preferred Grignard reagent to be used in the process of the invention is CH₃MgCl.
The Grignard reaction of the process of the invention can be performed in several solvents or mixtures of solvents, which are well known to the person skilled in the art, such as tetrahydrofuran, dimethoxyethane, diethyl ether, mono- and diglyme, toluene and the like. Preferred are tetrahydrofuran or diethyl ether or a mixture of these solvents. Tetrahydrofuran is the most preferred solvent.
The Grignard reagent is used equimolar or in excess to the steroid. Preferably, the molar ratio of the steroid to the Grignard reagent is 1:1 to 1:7.
The step wherein the trialkylsilyl protective group is introduced according to the process of the invention can be integrated in the 1,6-conjugate addition reaction, retaining the improved stereoselectivity in favor of the desired 7α-isomer and without loss of yield. The term "alkyl" used herein means a (1-4C)alkyl group, being a branched or unbranched alkyl group having 1 to 4 carbon atoms, in particular methyl or ethyl. The preferred protective groups are trimethylsilyl or triethylsilyl; most preferred is trimethylsilyl.

In the process of this invention a copper catalyst is used to catalyze the Grignard reaction, such as copper(II) acetate, copper(II) choride, copper(II) bromide, copper(II) iodide and the like. The preferred catalysts are selected from copper(II) acetate or copper(II) chloride. The most preferred catalyst is copper(II)acetate.
The reaction temperature, of the Grignard reaction of the process of the invention is not very critical, but should be kept low, preferably between -78 °C and 0 °C. The preferred temperature range is from -35 °C to -25 °C.
This new process for the first time makes a straightforward approach available for increasing the stereo selectivity of copper catalyzed 1,6-conjugate addition of methylmagnesiumhalogens to hydroxylated 4,6-unsaturated 3-ketosteroids. Consequently, laborious work-up procedures by troublesome chromatographic separations or by repetitive recrystallization are unnecessary.

In a suitable process of the invention a hydroxy 4,6-unsaturated 3-ketosteroid is treated with a trialkylsilyl reagent to protect the hydroxy functionality, after which a solution of the silyl protected steroid is added to a mixture of the copper catalyst and the Grignard reagent in an appropriate solvent, which after stirring for some time is followed by removal of the protective group. Removal of the trialkylsilyl protective group takes place under the typical conditions needed for the conjugation to the 4-unsaturated 3-ketosteroids, resulting in an effective one-step procedure. Suitably, the process is finalized by acid treatment for both removal of the trialkylsilyl group and equilibration of the 3,5-unsaturated magnesium-enolate to the desired 7α-methylsteroids of the formula 1.

The process of the invention is particularly useful for the preparation of 7α-methylsteroid (7a,17a)-17-hydroxy-7-methyl-19-norpregn-4-en-20-yn-3-one, suitable for preparation of (7α,17α)-17-hydroxy-7-methyl-19-norpregn-5(10)-en-20-yn-3-one (Tibolone), for the preparation of 7α-methyl-19-nortestosterone (MENT), and for the preparation of (7α)-21-hydroxy-7-methyl-19-norpregn-4-en-3-one, suitable for the preparation of 3-hydroxy-7α-methyl-21-[2'-methoxy-4'-(diethylaminomethyl)-phenoxy]-19-norpregna-1,3,5(10)triene citrate (SR 16234). For comparison, without introduction of a trialkylsilyl protective group, MENT was prepared by a copper catalyzed 1,6-conjugate addition from 17β-17-hydroxy-estra-4,6-diene-3-one in approximately 55% yield, with a 7β, β-ratio of 85:15 (according to procedures described in US 5,342,834; FR 4.521 M). Isolation of the 7α-isomer could only be achieved by repeated crystallizations from respectively heptane, and aqueous acetone, decreasing the overall yield to a moderate 44%. In a suitable process according to the invention, introduction of a trimethylsilyl protective group before the 1,6-conjugate addition reaction increases the overall yield of MENT to 79%, which is an improvement of the overall yield by 80%.

A further object of the present invention is the compound 21-hydroxy-19-norpregn-4,6-dien-3-one, which is a suitable intermedaite for use in the process of the present invention for the preparation of (7α)-21-hydroxy-7-methyl-19-norpregn-4-en-3-one, which in turn is suitable for the preparation of 3-hydroxy-7α-methyl-21-[2'-methoxy-4'-(diethylaminomethyl)-phenoxy]-19-norpregna-1,3,5(10)triene citrate (SR 16234).

The invention is further illustrated by the following examples, which does not mean any limitation.

### EXAMPLES

### EXAMPLE 1.

### 7α-methyl-19-nortestosterone (MENT).

Chlorotrimethylsilane (34.4 ml, 370 mmol) was added dropwise under nitrogen atmosphere at 20°C to a solution of (17β)-17-hydroxy-estra-4,6-diene-3-one (25 g, 92 mmol) (Wettstein, A. Helv.Chim.Acta, 1940;23: 388) and triethylamine (50 ml, 350 mmol) in anhydrous tetrahydrofuran (250 ml). The resulting mixture was stirred at reflux for 2 h. The reaction mixture was cooled to room temperature water (125 ml) was added and the mixture was extracted with toluene (250 ml). The combined organic layer was washed with an 10% aqueous sodium chloride solution (125 ml) and evaporated under reduced pressure to yield the 17-trimethylsilyloxy-intermediate as a residual solid, which without isolation was dissolved in anhydrous tetrahydrofuran (250 ml). This solution was added at -30°C in 2 h to a mixture of copper(II) acetate (2.58 g, 12.5 mmol) and a 3M solution of methylmagnesium chloride (61.6 g, 185 mmol) in tetrahydrofuran (120 ml), and the orange-red solution was stirred at -30°C for 1 h. Next the reaction mixture was poured into a solution of sulfuric acid (36.5 ml, 681 mmol) and water (500 ml) and stirred at 40°C for 2.5 h. Sodium acetate was added to a pH of 3.5, and the mixture was extracted with ethyl acetate (260 ml) and washed with aqueous ammonium chloride (83 ml, 10%). After removal of solvents by evaporation under reduced pressure aqueous acetone (150 ml, 50%) was added, and at -10°C 7α-methyl-19-nortestosterone (MENT) was crystallized (19.6 g, 79%) (in accordance to US 5,342,834; FR 4.521 M) in a 7a,β-ratio of 99:1. ¹H-NMR (400 MHz, CDCl₃): δ 5.68 (bt, 1H, C(4)H), 3.54 (t, 1H, C(17)H), 2.37-0.88 (m, 19H), 0.66 (s, 3H, C(18)H₃), 0.61 (d, 3H, C(7)CH₃).

### EXAMPLE 2.

### (7α,17α)-17-Hydroxy-7-methyl-19-norpregn-4-en-20-yn-3-one.

Chlorotrimethylsilane (6.28 ml, 67.5 mmol) was added dropwise under nitrogen atmosphere at 20°C to a solution of (17α)-17-hydroxy-19-norpregna-4,6-dien-20-yn-3-one (5.0 g, 17 mmol) (GB 935116) and pyridine (11.4 ml, 142 mmol) in anhydrous tetrahydrofuran (50 ml). The resulting mixture was stirred at reflux for 2.5 h. The reaction mixture was cooled to room temperature, water (25 ml) was added and the mixture was extracted with toluene (50 ml). The combined organic layer was washed with aqueous methanol (25 ml, 70%) and evaporated under reduced pressure to yield the 17-trimethylsilyloxy-intermediate as a residual solid, which without isolation was dissolved in anhydrous tetrahydrofuran (110 ml). This solution was added at - 30°C in 2 h to a mixture of copper(II) acetate (0.58 g, 2.9 mmol) and a 3M solution of methylmagnesium chloride (40 g, 118 mmol) in tetrahydrofuran (50 ml), and was stirred at - 30°C for 1 h. Next the reaction mixture was poured into a solution of sulfuric acid (6.8 ml, 126 mmol) and aqueous tetrahydrofuran (160 ml, 15%) and stirred at 20°C for 8 h. Sodium acetate was added to a pH of 3.5, and after removal of solvents by evaporation under reduced pressure (7α,17α)-17-hydroxy-7-methyl-19-norpregn-4-en-20-yn-3-one was crystallized (5.2 g, 99%) (in accordance to literature: Van Vliet, N.P., et al. Recl.Trav.Chim.Pays-Bas, 1986;105: 111), in a 7α,β-ratio of 95:5. ¹H-NMR (400 MHz, CDCl₃): δ 5.86 (bt, 1H, C(4)H), 2.59 (s, 1H, C(21)H), 2.52-0.86 (m, 19H), 0.94 (s, 3H, C(18)H₃), 0.79 (d, 3H, C(7)CH₃).

### EXAMPLE 3.

### 21-hydroxy-19-norpregn-4,6-dien-3-one.

4-Toluenesulfonic acid (100 mg, 0.56 mmol) was added under a nitrogen atmosphere at -5°C to a suspension of 21-hydroxy-19-norpregna-4-en-3-one (30.0 g, 99.2 mmol) (WO 01/58919) and triethyl orthoformiate (22.1 mL, 133 mmol) in ethanol (60 mL). After being stirred for 2 h at 0 °C the reaction mixture was quenched with triethylamine (0.22 mL, 1.6 mmol) and heated at reflux for 15 min. The reaction mixture was diluted with ethanol (50 mL), ethyl acetate (1.0 L), water (500 mL) and dichloromethane (500 mL). The organic layer was separated, the aqueous layer was extracted (dichloromethane, 2 x 250 mL) and the combined organic layers were concentrated *in vacuo.* A solution of the residue (50 g) in THF (140 mL) was added to a suspension of chloranil (26 g, 106 mmol) in methanol (140 mL), water (19 mL), acetic acid (5.1 mL) and pyridine (1.9 mL) at 20 °C. After being stirred at this temperature for 2 h, the reaction was quenched with a solution of sodium hydroxide (42 g, 1.05 mol) and sodium hydrosulfite (4.9 g, 28 mmol) in water (490 mL). The aqueous layer was extracted (dichloromethane, 3 x 500 mL), the combined organic layers were washed until pH neutral (water, 4 x 100 mL) and concentrated *in vacuo* to yield 21-hydroxy-19-norpregn-4,6-dien-3-one (25.0 g, 84%). The product was purified by column chromatography and crystallization from ethyl acetate (10.9 g, 37%). ¹H-NMR (400 MHz, CDCl₃): δ 6.25-6.17 (ABX dq, 2H, C(6)H and C(7)H), 5.76 (bd, 1H, C(4)H), 3.73-3.58 (m, 2H, C(21)H₂OH), 2.50-1.08 (m, 19H), 0.69 (s, 3H, C(18)H₃)

### (7α)-21-hydroxy-7-methyl-19-norpregn-4-en-3-one.

Chlorotrimethylsilane (2.0 mL, 33.3 mmol) was added dropwise under a nitrogen atmosphere at 20°C to a suspension of 21-hydroxy-19-norpregna-4,6-dien-3-one (5.0 g, 17 mmol) and triethylamine (12 mL, 80 mmol) in anhydrous tetrahydrofuran (100 mL). The resulting mixture was stirred at reflux for 1 h. The reaction mixture was cooled to room temperature, water (50 mL) was added, the organic layer was separated and the water layer was extracted with toluene (25 mL). The combined organic layers were concentrated *in vacuo,* yielding crude intermediate silylether (11 g). A solution of the silylether in tetrahydrofuran (121 mL) was added in 45 min to a solution of copper acetate (285 mg, 2.86 mmol) and methyl magnesium chloride (3.0 M solution in THF, 38.8 mL, 116 mmol) in THF (45 mL) at -20°C. After being stirred for 1 h at this temperature the reaction was quenched with a solution of sulfuric acid (6.7 g, 68 mmol) in water (125 mL) and THF (25 mL). After being stirred for 17 h at 20°C, the reaction mixture was distilled THF-free and the product was extracted with dichloromethane (3 x 50 mL). After concentration *in vacuo,* (7α)-21-hydroxy-7-methyl-19-norpregn-4-en-3-one was obtained as an amorphous solid (5.2 g, 99%) (in accordance to literature WO 01/58919) in a 7α/β ratio of 95:5. ¹H-NMR (400 MHz, CDCl₃): δ 5.85 (bt, 1H, C(4)H), 3.75-3.57 (m, 2H, C(21)H₂OH), 2.50-0.97 (m, 22H), 0.77 (d, 3H, C(7)CH₃), 0.67 (s, 3H, C(18)H₃).

## Claims

1. A process for the preparation of 7α-Methyl steroids of the formula I 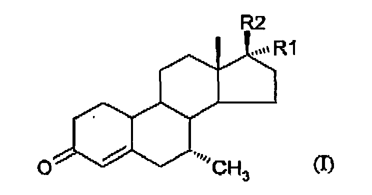
wherein R1 is hydrogen, methyl or C≡CH;
R2 is (CH₂)ₙOH, wherein n is 0, 1 or 2;
by a copper mediated 1,6-conjugate addition of a Grignard reagent CH₃MgX, X being a halogen, to the 4,6-unsaturated 3-ketosteroid of formula II, 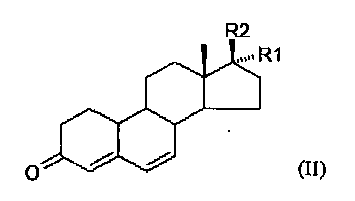 wherein R1 and R2 are as previously defined,
comprising protecting the hydroxy group of the steroid of formula II with a trialkylsilyl group, whereby the alkyl group is defined as being a branched or unbranched alkyl group having 1 to 4 carbon atoms. followed by treating the hydroxy protected steroid with the Grignard reagent.

2. The process of claim 1, wherein R1 is hydrogen, methyl, or C≡CH and R2 is OH.

3. The process of claim 1, wherein R1 is hydrogen and R2 is (CH₂)₂OH.

4. The process of any one of claims 1 to 3, wherein the Grignard reagent is CH₃MgCl.

5. The process of any one of claims 1 to 4, wherein the trialkylsilyl group is a trimethylsilyl group.

6. The process of any one of claims 1 to 5, wherein the solvent of the Grignard reaction is tetrahydrofuran, diethyl ether or a mixture thereof.

7. The process of any one of claims 1 to 6, wherein the concentration of the steroid is 0.1 to 0.3 molar.

8. The process of any one of claims 1 to 7, wherein the molar ratio of the steroid to the Grignard reagent is 1:1 to 1:7.

9. The process of any one of claims 1 to 8, wherein as copper catalyst copper(II) acetate or copper(II) chloride is used.

10. The process of any one of claims 1 to 9, wherein the reaction temperature of the Grignard reaction is -78 °C to 0 °C.

11. The compound 21-hydroxy-19-norpregn-4,6-dien-3-one.

## Patentansprüche

1. Verfahren zur Herstellung eines 7α-Methyl-Steroids der Formel I 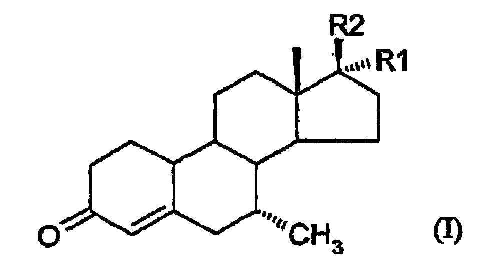 wobei
R1 Wasserstoff, Methyl oder C≡CH ist,
R2 (CH₂)ₙOH ist, wobei n 0, 1 oder 2 ist,
durch eine Kupfer-vermittelte 1,6-Konjugat-Hinzufügung eines Grignard-Reagenz CH₃MgX, wobei X ein Halogen ist, zu einem 4,6-ungesättigtem 3-Ketosteroid der Formel II 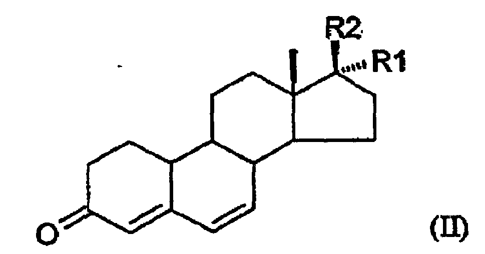 wobei R1 und R2 wie oben definiert sind,
umfassend das Schützen der Hydroxy-Gruppe des Steroids der Formel II mit einer Trialkylsilyl-Gruppe, wobei die Alkyl-Gruppe definiert ist als eine verzweigte oder unverzweigte Alkyl-Gruppe, die 1 bis 4 Kohlenstoffatome hat, gefolgt von der Behandlung des Hydroxy-geschützten Steroids mit dem Grignard-Reagenz.

2. Verfahren gemäss Anspruch 1, bei dem R1 Wasserstoff, Methyl oder C≡CH ist und bei dem R2 OH ist.

3. Verfahren gemäss Anspruch 1, bei dem R1 Wasserstoff ist und bei dem R2 (CH₂)₂OH ist.

4. Verfahren gemäss irgendeinem der Ansprüche 1 bis 3, bei dem das Grignard-Reagenz CH₃MgCl ist.

5. Verfahren gemäss irgendeinem der Ansprüche 1 bis 4, bei dem dir Trialkylsilyl-Gruppe eine Trimethylsilyl-Gruppe ist.

6. Verfahren gemäss irgendeinem der Ansprüche 1 bis 5, bei dem das Lösungsmittel der Grignard-Reaktion Tetrahydrofuran, Diethyläther oder eine Mischung davon ist.

7. Verfahren gemäss irgendeinem der Ansprüche 1 bis 6, bei dem die Konzentration des Steroids 0,1 bis 0,3 molar ist.

8. Verfahren gemäss irgendeinem der Ansprüche 1 bis 7, bei dem das molare Verhältnis des Steroids zum Grignard-Reagenz 1:1 zu 1:7 ist.

9. Verfahren gemäss irgendeinem der Ansprüche 1 bis 8, bei dem als Kupfer-Katalysator Kupfer(II)-Acetat oder Kupfer (II)- Chlorid eingesetzt wird.

10. Verfahren gemäss irgendeinem der Ansprüche 1 bis 9, bei dem die Reaktionstemperatur des Grignard-Reagenz zwischen -78° Celsius und 0° Celsius liegt.

11. Verbindung 21-Hydroxy-19-norpregn-4,6-dien-3-on.

## Revendications

1. Un procédé pour la préparation de 7α-méthylstéroïdes présentant la formule I 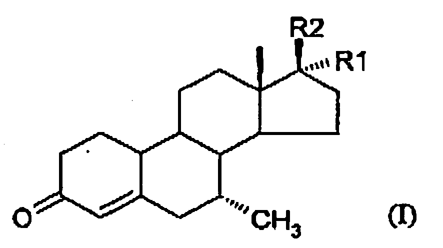 dans laquelle
R1 est un atome d'hydrogène, un radical méthyl ou un groupe C≡CH;
R2 est (CH₂)ₙOH, où n est 0, 1 ou 2;
par une addition 1,6-conjuguée, médiée par le cuivre, d'un réactif de Grignard de formule CH₃MgX, X étant un atome d'halogène, au 3-cétostéroïde insaturé en positions 4,6 de formule II 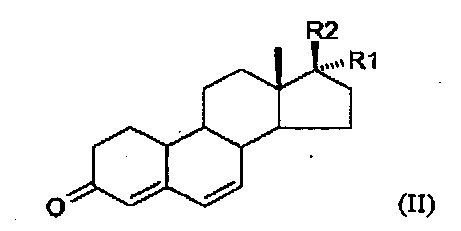 dans laquelle R1 et R2 sont tels que définis ci-dessus,
comprenant la protection du groupe hydroxy du stéroïde de formule II par un groupe trialkylsilyl, dans lequel le radical alkyl est défini comme étant un radical alkyl ramifié ou non ramifié comportant 1 à 4 atomes de carbone, suivie du traitement par le réactif de Grignard du stéroïde dont le groupe hydroxy.est protégé.

2. Le procédé selon la revendication 1, dans lequel R1 est un atome d'hydrogène, un radical méthyl, ou C≡CH et R2 est OH.

3. Le procédé selon la revendication 1, dans lequel R1 est un atome d'hydrogène et R2 est (CH₂)₂OH.

4. Le procédé selon une quelconque des revendications 1 à 3, dans lequel le réactif de Grignard est CH₃MgCl.

5. Le procédé selon une quelconque des revendications 1 à 4, dans lequel le groupe trialkylsilyl est un groupe triméthylsilyl.

6. Le procédé selon une quelconque des revendications 1 à 5, dans lequel le solvant de la réaction de Grignard est le tétrahydrofuranne, le diéthyl éther ou leurs mélanges.

7. Le procédé selon une quelconque des revendications 1 à 6, dans lequel la concentration du stéroïde est de 0,1 à 0,3 molaire.

8. Le procédé selon une quelconque des revendications 1 à 7, dans lequel le rapport molaire stéroïde/réactif de Grignard est de 1/1 à 1/7.

9. Le procédé selon une quelconque des revendications 1 à 8, dans lequel en tant que catalyseur à base de cuivre est employé de l'acétate de cuivre (II) ou du chlorure de cuivre (II).

10. Le procédé selon une quelconque des revendications 1 à 9, dans lequel la température de réaction de la réaction de Grignard est comprise entre -78°C et 0°C.

11. Le composé 21-hydroxy-19-norpregn-4,6-dien-3-one.
